# EUROPEAN PATENT SPECIFICATION

(11) **EP 2 921 196 B1**
(45) Date of publication and mention of the grant of the patent: **28.12.2016**
(21) Application number: 15158260.8
(22) Date of filing: 09.03.2015
(51) Int. Cl.: A61M 25/09

(54) **Guide wire**
Führungsdraht
Guide-fil

(30) Priority: 20.03.2014 JP 2014057516
(43) Date of publication of application: 23.09.2015
(73) Proprietor: ASAHI INTECC CO., LTD., Nagoya-shi, Aichi 463-0024 (JP)
(72) Inventor: MATSUO, Kenichi c/o ASAHI INTECC CO., LTD., Nagoya-shi, Aichi 463-0024 (JP)
(74) Representative: Winter, Brandl, Fürniss, Hübner, Röss, Kaiser, Polte - Partnerschaft mbB

(56) References cited:
- EP-A1- 1 266 670
- EP-A1- 1 938 859
- US-A- 5 507 301
- US-A1- 2003 181 828

## Description

### [Technical Field]

The present invention relates to a guide wire inserted into a lumen such as a blood vessel.

### [Background Art]

Guide wires used for insertion of catheters into blood vessels are known. In the insertion of a catheter, a guide wire is first inserted into a blood vessel and then the catheter is moved along the guide wire. Thus, the guide wire acts as a guide for guiding the catheter to a lesion.

The guide wire includes a core shaft and a coil body covering the core shaft. The coil body of the guide wire may include two kinds of connected coils. The two coils are connected by interposing the loops or turns of one coil between the loops or turns of the other coil (For example, Patent Literature 1, Patent Literature 2, and Patent Literature 3). According to the connecting method, a coil restoring force can hold the turns of one of the coils between the turns of the other coil. Thus, even before a connecting portion is bonded with a brazing metal, the central axes of the two coils are easily kept aligned with each other, advantageously facilitating an assembly operation of the guide wire.

### [Citation List]

### [Patent Literatures]

[Patent Literature 1] Japanese Unexamined Patent Application Publication No. S60-168466
[Patent Literature 2] Japanese Unexamined Patent Application Publication No. 2005-46603
[Patent Literature 3] Japanese Unexamined Patent Application Publication No. 2006-297152

### [Technical Problem]

In the guide wire of Patent Literature 2 or Patent Literature 3, however, the coil turns in a connecting portion do not have a clearance interposed between them. This makes it difficult to apply a brazing metal into the connecting portion so as to obtain sufficient bonding strength. In the guide wire of Patent Literature 1, a large pitch is set between two coils in a connecting portion so as to provide a clearance between coil turns on the connecting portion. However, this makes it difficult to keep aligned the central axes of the coils before brazing, leading to poor workability in the connection of the two coils.

As described above, in the related art, it is difficult to obtain both workability in the connection of the two coils and ease of application of brazing metal into the connecting portion of the coils.

Document US 2003/181828 A1 describes a guidewire 1 with a core 23, two helical coils 2, 3, a connection between the two coils at a joint section 33 where both windings are intermeshing with one another.

### [Summary of Invention]

The present, invention has been devised to solve the above problem of the related art. An object of the present invention is to provide a technique that can obtain both workability in the connection of two coils and ease of application of brazing metal into the connecting portion of the two coils in a guide wire having a coil body composed of the two connected coils.

### [Solution to Problem]

In order to solve the problem, the guide wire of the present invention is configured as follows: the guide wire including: a core shaft; and a coil body that covers the core shaft and includes a distal-end coil and a proximal-end coil, wherein the distal-end coil has a plurality of turns being arranged axially in succession and including in the order from a proximal end towards a distal end of the distal end coil a at least first turn, a second turn and a third turn and the proximal coil has a plurality of turns being arranged axially in succession and including in the order from a distal end towards a proximal end of the proximal-end coil at least a first turn and a second turn. The distal-end coil and the proximal-end coil are connected to each other with alternately disposed turns, the proximal-end coil having the first turn that is held from both sides or clamped from both sides by the second turn and third turn of the distal-end coil and the second turn that is in contact with a distal-end side of the first turn of the distal-end coil. According to the present invention, a gap is provided between the second turn of the proximal-end coil and the second turn of the distal-end coil.

In the guide wire of the present invention, the first turn of the proximal-end coil is held or clamped by the by the second turn and third turn of the distal-end coil from both sides and the second turn adjacent to the first turn of the proximal-end coil is in contact with a distal-end side of the first turn of the distal-end coil. Hence, even before a connecting portion is bonded with brazing metal or the like, the central axes of the two coils can be easily kept aligned with each other. This facilitates the connection of the coils (an assembly operation of the guide wire).

The guide wire of the present invention has a gap between the second turn of the proximal-end coil and the second turn of the distal-end coil. This facilitates the application of bonding material e.g. brazing metal or the like into the connecting portion, sufficiently securing bonding strength between the distal-end coil and the proximal-end coil and bonding strength between these coils and a core shaft. Therefore, the guide wire of the present invention may include bonding material which is applied into the connecting portion of the distal-end coil and the proximal-end coil via the gap, thereby joining the core shaft and the coil body to each other. Preferentially, brazing metal is used as a bonding material.

Further, the proximal-end coil and/or the distal-end coil may be formed of a single wire or a stranded wire comprised of a plurality of single wires.

Further, the number of turns of each coil and the specific coil geometry including pitch and turning angle can be varied depending on a desired application of the guide wire.

Furthermore, more than one gap can be provided between the interposed turns of the proximal-end coil and the distal-end coil. The proximal-end coil may include a third turn and an additional gap may be provided between the third turn of the proximal-end coil and the first turn of the distal-end coil, preferentially in addition to the gap between the second turn of the proximal-end coil and the second turn of the distal-end coil.

As described above, the guide wire of the present invention can obtain both workability in the connection of the two coils and ease of application of the brazing metal into the connecting portion of the coils.

In the guide wire of the present invention, a wire forming the proximal-end coil, i.e. the plurality turns of the proximal-end coil, may have a larger wire diameter than a wire forming the distal-end coil, i.e. the plurality of turns of the distal-end coil.

In the guide wire of the present invention, the wire of the proximal-end coil is larger in diameter than the wire of the distal-end coil, thereby preventing the restoring force of the distal-end coil (a force of restoring the original shape of the extended coil) from deforming the proximal-end coil. Thus, in the connecting portion of the two coils, the gap can be easily obtained between the wire of the distal-end coil and the wire of the proximal-end coil.

In the guide wire of the present invention, the proximal-end coil may be made of a material having a larger modulus of elasticity than the distal-end coil.

In the guide wire of the present invention, the proximal-end coil is made of a material having a larger modulus of elasticity than the distal-end coil, thereby preventing the restoring force of the distal-end coil from deforming the proximal-end coil. Even if the distal-end coil and the proximal-end coil are difficult to vary in wire diameter (For example, in the design of a guide wire having an extremely small outside diameter) for design reasons, in particular, it is possible to prevent the restoring force of the distal-end coil from deforming the proximal-end coil. Thus, in the connecting portion of the two coils, the gap between the wires of the distal-end coil and the proximal-end coil can be easily and reliably obtained.

### [Brief Description of Drawings]

[FIG. 1] FIG. 1 is an explanatory drawing showing the configuration of a guide wire according to a first embodiment of the present invention.
[FIG. 2] FIG. 2 is an enlarged view of the connecting portion of two coils in the guide wire according to the first embodiment of the present invention.
[FIG. 3] FIG. 3 shows explanatory drawings illustrating a part of an assembly operation of the guide wire according to the first embodiment of the present invention: FIG. 3(a) shows that the distal-end coil is brazed to the front end of a core shaft, FIG. 3(b) shows that the distal-end coil and the proximal-end coil are connected to each other with the interposed turns, and FIG. 3(c) shows a connecting portion brazed between the distal-end coil and the proximal-end coil.
[FIG. 4] FIG. 4 is an enlarged view of a connecting portion between two coils in a guide wire according to a second embodiment of the present invention.
[FIG. 5] FIG. 5 is an enlarged view showing the connecting portion of two coils in a guide wire according to a third embodiment of the present invention.

### [Description of Embodiments]

### A. First Embodiment:

For clarification of the contents of the present invention, embodiments of a guide wire according to the present invention will be described below.

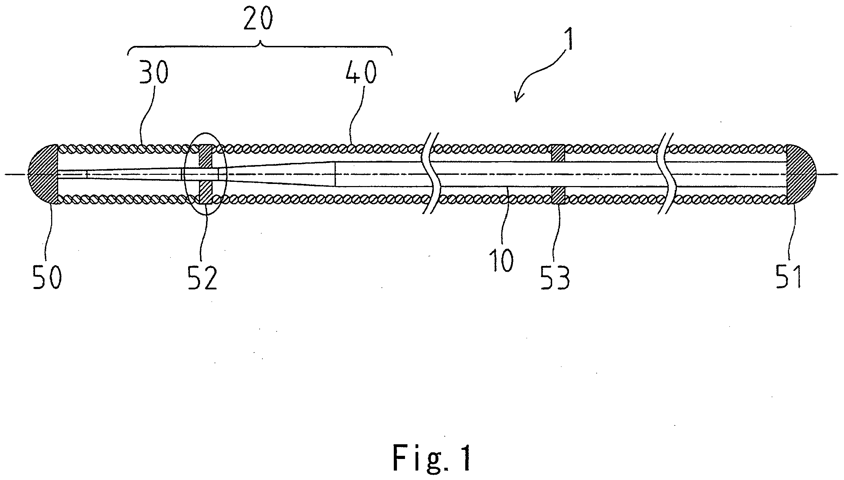

FIG. 1 is an explanatory drawing showing the configuration of a guide wire 1 according to a first embodiment. As shown in FIG. 1, the guide wire 1 of the present embodiment includes a core shaft 10 and a coil body 20 covering the core shaft 10.

The core shaft 10 is tapered down toward the front end of the guide wire 1 to have flexibility on the front end of the guide wire 1. The coil body 20 includes two connected coils (a distal-end coil 30 and a proximal-end coil 40). Each of the two coils is formed of a single wire having a plurality of turns being arranged axially in succession, wherein the plurality of turns of the distal-end coil 30 include in the order from a proximal end towards a distal end of the distal-end coil 30 (at least) a first turn 30c, a second turn 30b and a third turn 30a, and the plurality of turns of the proximal-end coil 40 include in the order from a distal end towards a proximal end of the proximal-end coil 40 (at least) a first turn 40a, a second turn 40b and a third turn 40c.

The front end of the core shaft 10 and the front end of the coil body 20 are joined to each other via a front-end joint 50. The rear end of the core shaft 10 and the rear end of the coil body 20 are joined to each other via a rear-end joint 51. Moreover, the core shaft 10 and the coil body 20 are joined to each other via a first intermediate joint 52 on the connecting point of the distal-end coil 30 and the proximal-end coil 40 and via a second intermediate joint 53 on an intermediate portion of the proximal-end coil 40.

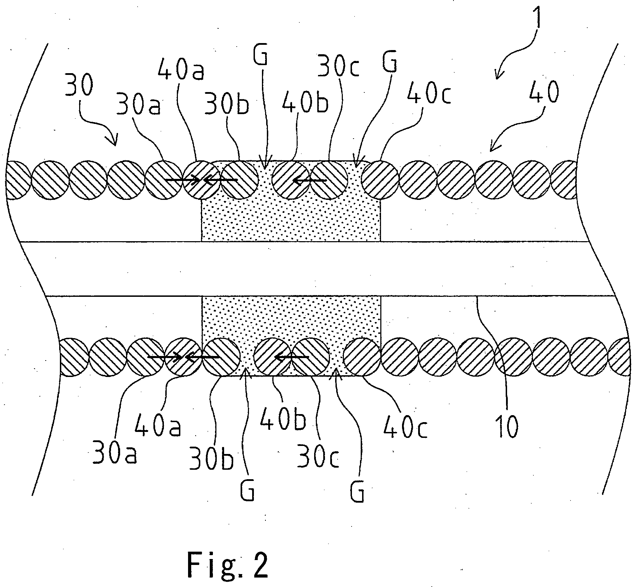

FIG. 2 is an enlarged view of the connecting portion of the two coils in the guide wire 1 according to the first embodiment of the present invention. As shown in FIG. 2, in the present embodiment, the two coils (the distal-end coil 30 and the proximal-end coil 40) are connected to each other by alternately disposing (interposing) the wire turns of the distal-end coil 30 and the wire turns of the proximal-end coil 40.

In the guide wire 1 of the present embodiment, the first turn 40a at the front or distal end of the proximal-end coil 40 is held from both sides or clamped between the second turn 30b and third turn 30a of the distal-end coil 30. The second turn 40b of the proximal-end coil 40 is in contact with the first turn 30c of the distal-end coil 30, the first turn 30c being located on the proximal-end side of the second turn 40b of the proximal-end coil 40. Furthermore, in the connecting portion of the two coils, a gap G is provided between the second turn 40b of the proximal-end coil 40 and the second turn 30b of the distal-end coil 30, the second turn 30b being adjacent to the distal-end side of the second turn 40b of the proximal-end coil 40.

In the present embodiment, the gap G is also provided between the third turn 40c of the proximal-end coil 40 and the first turn 30c of the distal-end coil 30, the turn 30c being adjacent to the distal-end side of the third turn 40c of the proximal-end coil 40.

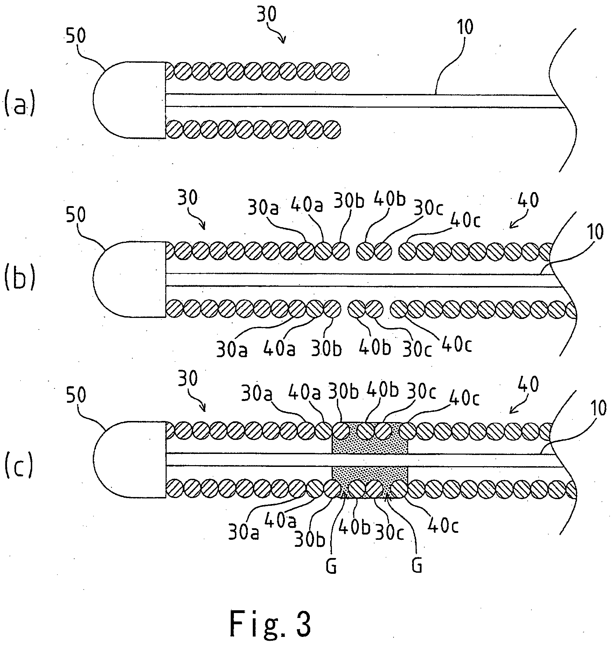

FIG. 3 shows explanatory drawings illustrating a part of an assembly operation of the guide wire 1 according to the first embodiment of the present invention. FIG. 3(a) shows that the distal-end coil 30 is brazed to the front end of the core shaft 10. FIG. 3(b) shows that the distal-end coil 30 and the proximal-end coil 40 are connected to each other with the interposed turns. FIG. 3 (c) shows a connecting portion brazed between the distal-end coil 30 and the proximal-end coil 40.

As shown in FIG. 3 (a), in the guide wire 1 of the first embodiment, the front ends of the core shaft 10 and the distal-end coil 30 are brazed to form the front-end joint 50. Subsequently, as shown in FIG. 3(b), the proximal-end coil 40 is inserted from the proximal end of the core shaft 10, and then the proximal end of the distal-end coil 30 and the distal end of the proximal-end coil 40 are connected to each other with the interposed turns.

In the guide wire 1 of the present embodiment, the distal end of the proximal-end coil 40 has a relatively large pitch with respect to the proximal end of the distal-end coil 30 before the connection. Thus, when the two coils are connected, the first turn 40a of the proximal-end coil 40 is held by or clamped between the third turn 30a and second turns 30b of the distal-end coil 30 from both sides and the second turn 40b adjacent to the proximal-end side of the first turn 40a is in contact with the third turn 30c of the distal-end coil 30 from the proximal-end side of the second turn 40b (See FIG. 3(b)). Hence, even before brazing, the central axes of the two coils are easily kept aligned with each other. This can eliminate the need for, for example, tentative fastening for fixing the central axes of the two coils, facilitating the connection of the coils (an assembly operation of the guide wire 1 as well).

After the distal-end coil 30 and the proximal-end coil 40 are connected as shown in FIG. 3(b), brazing metal is applied to the connecting portion.

As described above, in the guide wire 1 of the present embodiment, the distal end of the proximal-end coil 40 has a relatively large pitch with respect to the proximal end of the distal-end coil 30 before the connection. Thus, while the two coils are connected, the gap G is formed between the second turn 40b of the proximal-end coil 40 and the second turn 30b of the distal-end coil 30, the second turn 30b being adjacent to the distal-end side of the second turn 40b of the proximal-end coil 40. Moreover, the gap G is formed between the third turn 40c of the proximal-end coil 40 and the first turn 30c of the distal-end coil 30, the first turn 30c being adjacent to the distal-end side of the third turn 40c. Thus, as shown in FIG. 3(c), brazing metal enters from the gap G so as to form the first intermediate joint 52. This can sufficiently obtain bonding strength between the distal-end coil 30 and the proximal-end coil 40 and bonding strength between these coils and the core shaft 10.

As described above, the guide wire 1 of the present invention can obtain both workability in the connection of the two coils (the distal-end coil 30 and the proximal-end coil 40) and ease of application of brazing metal into the connecting portion of the coils.

### B. Second Embodiment:

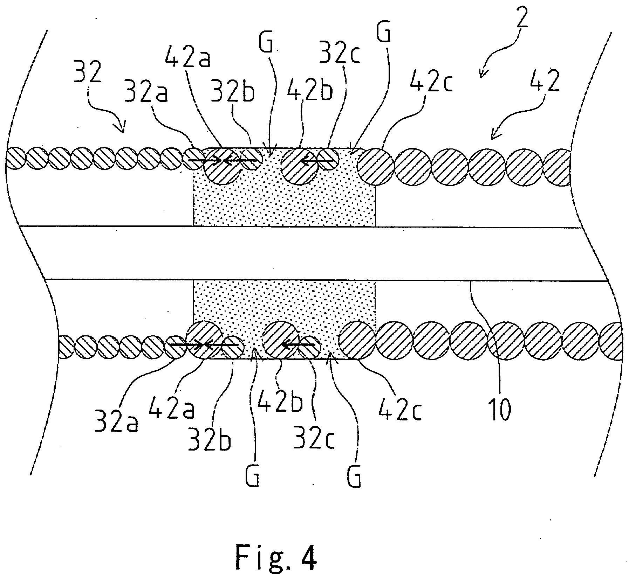

FIG. 4 is an enlarged view of a connecting portion between two coils in a guide wire 2 according to a second embodiment of the present invention. The guide wire 2 of the second embodiment is different from the guide wire 1 of the first embodiment as follows: in the guide wire 2 of the second embodiment, a wire forming a proximal-end coil 42 is larger in diameter than a wire forming a distal-end coil 32.

Other configurations of the guide wire 2 according to the second embodiment are identical to those of the guide wire 1 according to the first embodiment. Specifically, a first turn 42a of the proximal-end coil 42 is held by or clamped between the third turn 32a and third turn 32b of the distal-end coil 32 from both sides, and a second turn 42b of the proximal-end coil 42, adjacent to the proximal-end side of the first turn 42a is in contact with the first turn 32c of the distal-end coil 32 from the proximal-end side of the second turn 42b. Moreover, in the connecting portion of the two coils, a gap G is provided between the second turn 42b of the proximal-end coil 42 and the second turn 32b of the distal-end coil 32, the second turn 32b being adjacent to the distal-end side of the second turn 42b. The gap G is also provided between the third turn 42c of the proximal-end coil 42 and the first turn 32c of the distal-end coil 32, the first turn 32c being adjacent to the distal-end side of the third turn 42c.

The guide wire 2 of the present embodiment can also obtain both workability in the connection of the two coils (the distal-end coil 32 and the proximal-end coil 42) and ease of application of brazing metal into the connecting portion of the coils.

The wire of the proximal-end coil 42 is larger in diameter than the wire of the distal-end coil 32, thereby preventing the restoring force of the distal-end coil 32 (a force of restoring the original shape of the extended coil) from deforming the proximal-end coil 42. Thus, in the connecting portion of the two coils, the gap can be easily obtained between the wire of the distal-end coil 32 and the wire of the proximal-end coil 42.

### C. Third Embodiment:

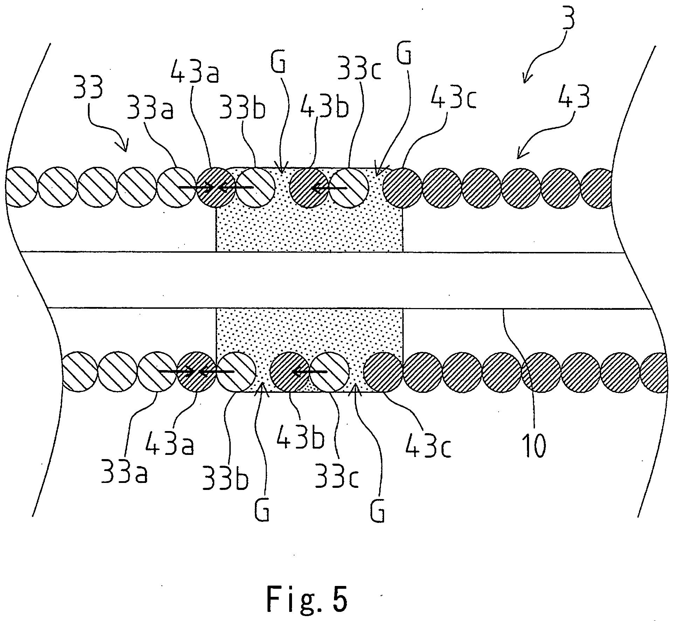

FIG. 5 is an enlarged view showing the connecting portion of two coils in a guide wire 3 according to a third embodiment of the present invention. The guide wire 3 of the third embodiment shown is different from the guide wires of the foregoing embodiments as follows: in the guide wire 3 of the third embodiment, a proximal-end coil 43 is made of a material having a larger modulus of elasticity than the distal-end coil 33.

In the present embodiment, the distal-end coil 33 is made of platinum while the proximal-end coil 43 is made of stainless steel having a larger modulus of elasticity than platinum.

Other configurations of the guide wire 3 according to the third embodiment are identical to those of the guide wire 1 according to the first embodiment (or the guide wire 2 according to the second embodiment). Specifically, a first turn 43a of the proximal-end coil 43 is held by or clamped between a third turn 33a and turn 33b of the distal-end coil 33 from both sides and a second turn 43b adjacent to the proximal-end side of the first turn 43a is in contact with a first turn 33c of the distal-end coil 33 from the proximal-end side of the second turn 43b. Moreover, in the connecting portion of the two coils, a gap G is provided between the second turn 43b of the proximal-end coil 43 and the second turn 33b of the distal-end coil 33 on the distal-end side of the second turn 43b. The gap G is also provided between the third turn 43c of the proximal-end coil 43 and the first turn 33c of the distal-end coil 33 on the distal-end side of the third turn 43c.

The guide wire 3 of the present embodiment can obtain both workability in the connection of the two coils (the distal-end coil 33 and the proximal-end coil 43) and ease of application of brazing metal into the connecting portion of the coils, like the guide wires of the first and second embodiments.

The proximal-end coil 43 is made of a material having a larger modulus of elasticity than the distal-end coil 33, thereby preventing the restoring force of the distal-end coil 33 from deforming the proximal-end coil 43. Even if the distal-end coil 33 and the proximal-end coil 43 are difficult to vary in wire diameter (For example, in the design of a guide wire having an extremely small outside diameter) for design reasons, in particular, it is possible to prevent the restoring force of the distal-end coil 33 from deforming the proximal-end coil 43. Thus, in the connecting portion of the two coils, the gap between the wires of the distal-end coil 33 and the proximal-end coil 43 can be easily and reliably obtained.

The guide wires according to the embodiments of the present invention were described in the above explanation. The present invention is not limited to these embodiments and can be implemented in various forms within the scope of the invention.

For example, in the guide wire 3 of the third embodiment, the proximal-end coil 43 is made of a material having a larger modulus of elasticity than the distal-end coil 33 (See FIG. 5). In FIG. 5, the wire of the distal-end coil 33 and the wire of the proximal-end coil 43 are identical in diameter. However, the wire of the proximal-end coil may be larger in diameter than the wire of the distal-end coil (not shown).

This configuration includes the proximal-end coil made of a material having a larger modulus of elasticity than the distal-end coil, thereby more reliably preventing deformation of the proximal-end coil. Hence, in the connecting portion of the two coils, the gap between the turns of the distal-end coil and the proximal-end coil can be obtained with higher reliability, thereby sufficiently securing bonding strength between the two coils (the distal-end coil and the proximal-end coil) and bonding strength between these coils and a core shaft.

### [Reference Signs List]

- 1, 2, 3 ...: guide wire
- 10 ...: core shaft
- 20 ...: coil body
- 30, 32, 33 ...: distal-end coil
- 30a, 32a, 33a ...: (distal-end coil) third turn
- 30b, 32b, 33b ...: (distal-end coil) second turn
- 30c, 32, 33c ...: (distal-end coil) first turn
- 40, 42, 43 ...: proximal-end coil
- 40a, 42a, 43a ...: (proximal-end coil) first turn
- 40b, 42b, 43b ...: (proximal-end coil) second turn
- 40c, 42c, 43c ...: (proximal-end coil) third turn
- 50 ...: front-end joint
- 51 ...: rear-end joint
- 52 ...: first intermediate joint
- 53 ...: second intermediate joint

## Claims

1. A guide wire (1; 2; 3) comprising:
a core shaft (10); and
a coil body (20) that covers the core shaft (10) and includes a distal-end coil (30; 32; 33) and a proximal-end coil (40; 42; 43), wherein
the distal-end coil (30; 32; 33) has a plurality of turns (30a, 30b, 30c) being arranged axially in succession and including in the order from a proximal end towards a distal end of the distal-end coil (30; 32; 33) at least a first turn (30c), a second turn (30b) and a third turn (30a),
the proximal-end coil (40; 42; 43) has a plurality of turns (40a, 40b, 40c) being arranged axially in succession and including in the order from a distal end towards a proximal end of the proximal-end coil (40; 42; 43) at least a first turn (40a) and a second turn (40b), and
the distal-end coil (30; 32; 33) and the proximal-end coil (40; 42; 43) are connected to each other with alternately disposed turns (30a, 30b, 30c, 40a, 40b, 40c; 32a, 32b, 32c, 42a, 42b, 42c; 33a, 33b, 33c, 43a, 43b, 43c) such that the first turn (40a; 42a; 43a) of the proximal-end coil (40; 42; 43) is clamped by the second turn (30b; 32b; 33b) and third turn (30a; 32a; 33a) of the distal-end coil (30; 32; 33), and the second turn (40b; 42b; 43b) of the proximal-end coil (40; 42, 43) is in contact with a distal-end side of the first turn (30c; 32c; 33c) of the distal-end coil (30; 32; 33), **characterized in that**
a gap (G) is provided between the second turn (40b; 42b; 43b) of the proximal-end coil (40; 42; 43) and the second turn (30b; 32b; 33b) of the distal-end coil (30; 32; 33).

2. The guide wire (2) according to claim 1, wherein a wire diameter of a wire forming the proximal-end coil (42) is larger than a wire diameter of a wire forming the distal-end coil (32).

3. The guide wire (3) according to claim 1 or 2, wherein the proximal-end coil (43) is made of a material having a larger modulus of elasticity than the distal-end coil (33).

4. The guide wire (1; 2; 3) according to any one of claims 1 to 3, wherein
the core shaft (10) and the coil body (20) are joined to each other by bonding material applied into the connecting portion of the distal-end coil (30; 32; 33) and the proximal-end coil (40; 42; 43) via the gap (G).

5. The guide wire (1; 2; 3) according to claim 4, wherein
the core shaft (10) and the coil body (20) are joined to each other by brazing metal applied into the connecting portion of the distal-end coil (30; 32; 33) and the proximal-end coil (40; 42; 43) via the gap (G).

6. The guide wire (1; 2; 3) according any one of claims 1 to 5, wherein
the plurality of turns (40a, 40b, 40c; 42a, 42b, 42c; 43a, 43b, 43c) of the proximal-end coil (40; 42; 43) further include a third turn (40c; 42c; 43c), and
a gap (G) is also provided between the third turn (40c) of the proximal-end coil (40; 42; 43) and the first turn (30c; 32c; 33c) of the distal-end coil (30; 32; 33).

## Patentansprüche

1. Führungsdraht (1; 2; 3) mit:
einem Kernschaft (10); und
einem Wicklungskörper (20), der den Kernschaft (10) umgibt und eine distale Wicklung (30; 32; 33) und eine proximale Wicklung (40; 42; 43) umfasst, wobei
die distale Wicklung (30; 32; 33) eine Vielzahl von Wicklungsgängen (30a, 30b, 30c) hat, die axial nacheinander angeordnet sind und vom proximalen Ende in Richtung des distalen Endes der distalen Wicklung (30; 32; 33) wenigstens einen ersten Wicklungsgang (30c), einen zweiten Wicklungsgang (30b) und einen dritten Wicklungsgang (30a) in Folge aufweisen,
die proximale Wicklung (40; 42; 43) eine Vielzahl von Wicklungsgängen (40a, 40b, 40c) hat, die axial nacheinander angeordnet sind und vom distalen Ende in Richtung des proximalen Endes der proximalen Wicklung (40; 42; 43) wenigstens einen ersten Wicklungsgang (40a) und einen zweiten Wicklungsgang (40b) in Folge aufweisen, und
die distale Wicklung (30; 32; 33) und die proximale Wicklung (40; 42; 43) durch abwechselnd angeordnete Wicklungsgänge (30a, 30b, 30c, 40a, 40b, 40c; 32a, 32b, 32c, 42a, 42b, 42c; 33a, 33b, 33c, 43a, 43b, 43c) in der Weise miteinander verbunden sind, dass der erste Wicklungsgang (40a; 42a; 43a) der proximalen Wicklung (40; 42; 43) zwischen dem zweiten Wicklungsgang (30b; 32b; 33b) und dritten Wicklungsgang (30a; 32a; 33a) der distalen Wicklung (30; 32; 33) eingeklemmt und der zweite Wicklungsgang (40b; 42b; 43b) der proximalen Wicklung (40; 42, 43) in Kontakt mit der distalen Seite des ersten Wicklungsgangs (30c; 32c; 33c) der distalen Wicklung (30; 32; 33) ist, **dadurch gekennzeichnet, dass**
zwischen dem zweiten Wicklungsgang (40b; 42b; 43b) der proximale Wicklung (40; 42; 43) und dem zweiten Wicklungsgang (30b; 32b; 33b) der distalen Wicklung (30; 32; 33) ein Abstand (G) vorgesehen ist.

2. Führungsdraht (2) nach Anspruch 1, wobei der Drahtdurchmesser eines die proximale Wicklung (42) bildenden Drahts größer ist als der Drahtdurchmesser eines die distale Wicklung (32) bildenden Drahts.

3. Führungsdraht (3) nach Anspruch 1 oder 2, wobei die proximale Wicklung (43) aus einem Material mit einem höheren Elastizitätsmodul hergestellt ist als die distale Wicklung (33).

4. Führungsdraht (1; 2; 3) nach einem der Ansprüche 1 bis 3, wobei
der Kernschaft (10) und der Wicklungskörper (20) durch ein über den Abstand (G) in den Verbindungsbereich der distalen Wicklung (30; 32; 33) und der proximalen Wicklung (40; 42; 43) aufgebrachtes Fügematerial aneinandergefügt sind.

5. Führungsdraht (1; 2; 3) nach Anspruch 4, wobei
der Kernschaft (10) und der Wicklungskörper (20) durch ein über den Abstand (G) in den Verbindungsbereich der distalen Wicklung (30; 32; 33) und der proximalen Wicklung (40; 42; 43) aufgebrachtes Lötmetall aneinandergefügt sind.

6. Führungsdraht (1; 2; 3) nach einem der Ansprüche 1 bis 5, wobei
die Vielzahl von Wicklungsgängen (40a, 40b, 40c; 42a, 42b, 42c; 43a, 43b, 43c) der proximalen Wicklung (40; 42; 43) des Weiteren einen dritten Wicklungsgang (40c; 42c; 43c) aufweist, und
auch zwischen dem dritten Wicklungsgang (40c) der proximalen Wicklung (40; 42; 43) und dem ersten Wicklungsgang (30c; 32c; 33c) der distalen Wicklung (30; 32; 33) ein Abstand (G) vorgesehen ist.

## Revendications

1. Guide-fil (1; 2; 3), comprenant:
un arbre central (10); et
un corps de bobine (20) qui recouvre l'arbre central (10) et qui comprend une bobine d'extrémité distale (30; 32; 33) et une bobine d'extrémité proximale (40; 42; 43), dans lequel
la bobine d'extrémité distale (30; 32; 33) présente une pluralité de spires (30a, 30b, 30c) qui sont agencées axialement de façon successive et qui comprennent dans l'ordre à partir d'une extrémité proximale en direction d'une extrémité distale de la bobine d'extrémité distale (30; 32; 33) au moins une première spire (30c), une deuxième spire (30b) et une troisième spire (30a),
la bobine d'extrémité proximale (40; 42; 43) présente une pluralité de spires (40a, 40b, 40c) qui sont agencées axialement de façon successive et qui comprennent dans l'ordre à partir d'une extrémité distale en direction d'une extrémité proximale de la bobine d'extrémité proximale (40; 42; 43) au moins une première spire (40a) et une deuxième spire (40b), et
la bobine d'extrémité distale (30; 32; 33) et la bobine d'extrémité proximale (40; 42; 43) sont connectées l'une à l'autre avec des spires disposées alternativement (30a, 30b, 30c, 40a, 40b, 40c; 32a, 32b, 32c, 42a, 42b, 42c; 33a, 33b, 33c, 43a, 43b, 43c) de telle sorte que la première spire (40a; 42a; 43a) de la bobine d'extrémité proximale (40; 42; 43) soit serrée par la deuxième spire (30b; 32b; 33b) et par la troisième spire (30a; 32a; 33a) de la bobine d'extrémité distale (30; 32; 33), et que la deuxième spire (40b; 42b; 43b) de la bobine d'extrémité proximale (40; 42; 43) soit en contact avec un côté d'extrémité distale de la première spire (30c; 32c; 33c) de la bobine d'extrémité distale (30; 32; 33),
**caractérisé en ce que**:
un espace (G) est prévu entre la deuxième spire (40b; 42b, 43b) de la bobine d'extrémité proximale (40; 42; 43) et la deuxième spire (30b; 32b; 33b) de la bobine d'extrémité distale (30; 32; 33).

2. Guide-fil (2) selon la revendication 1, dans lequel un diamètre de fil d'un fil formant la bobine d'extrémité proximale (42) est plus grand qu'un diamètre de fil d'un fil formant la bobine d'extrémité distale (32).

3. Guide-fil (3) selon la revendication 1 ou 2, dans lequel la bobine d'extrémité proximale (43) est constituée d'un matériau dont le module d'élasticité est plus élevé que celui de la bobine d'extrémité distale (33).

4. Guide-fil (1; 2; 3) selon l'une quelconque des revendications 1 à 3, dans lequel l'arbre central (10) et le corps de bobine (20) sont joints l'un à l'autre par un matériau de liaison qui est appliqué dans la partie de connexion de la bobine d'extrémité distale (30; 32; 33) et de la bobine d'extrémité proximale (40; 42; 43) par l'intermédiaire de l'espace (G).

5. Guide-fil (1; 2; 3) selon la revendication 4, dans lequel l'arbre central (10) et le corps de bobine (20) sont joints l'un à l'autre par un métal de brasage qui est appliqué dans la partie de connexion de la bobine d'extrémité distale (30; 32; 33) et de la bobine d'extrémité proximale (40; 42; 43) par l'intermédiaire de l'espace (G).

6. Guide-fil (1; 2; 3) selon l'une quelconque des revendications 1 à 5, dans lequel:
la pluralité de spires (40a, 40b, 40c; 42a, 42b, 42c; 43a, 43b, 43c) de la bobine d'extrémité proximale (40; 42; 43) comprend en outre une troisième spire (40c; 42c; 43c), et
un espace (G) est également prévu entre la troisième spire (40c) de la bobine d'extrémité proximale (40; 42; 43) et la première spire (30c; 32c; 33c) de la bobine d'extrémité distale (30; 32; 33).
